# EUROPÄISCHE PATENTSCHRIFT

(11) **EP 1 012 156 B1**
(45) Veröffentlichungstag und Bekanntmachung des Hinweises auf die Patenterteilung: **10.09.2008**
(21) Anmeldenummer: 98948944.8
(22) Anmeldetag: 09.09.1998
(51) Int. Cl.: C07H 21/00

(54) **NUCLEINSÄURE-SEQUENZEN UND VERFAHREN ZUM NACHWEIS VON BAKTERIEN DER GATTUNG PSEUDOMONAS AERUGINOSA**
NUCLEIC ACID SEQUENCES AND METHOD FOR ISOLATING BACTERIES FROM THE TYPE GENUS PSEUDOMONAS AERUGINOSA
SEQUENCES D'ACIDES NUCLEIQUES ET PROCEDE DE MISE EN EVIDENCE DE BACTERIES DU GENRE DES PSEUDOMONAS AERUGINOSA

(30) Priorität: 09.09.1997 DE 19739611
(43) Veröffentlichungstag der Anmeldung: 28.06.2000
(73) Patentinhaber: Biotecon Diagnostics GmbH, 10589 Berlin (DE)
(72) Erfinder: BERGHOF, Kornelia, D-12355 Berlin (DE); GASCH, Alexander, D-13359 Berlin (DE); BRÄUER, Anja, D-13409 Berlin (DE); GRÖNEWALD, Cordt, D-10555 Berlin (DE); WILBORN, Freimut, D-14057 Berlin (DE); ROLFS, Arndt, D-18055 Rostock (DE)
(74) Vertreter: Boeters, Hans Dietrich
(86) Internationale Anmeldenummer: PCT/EP1998/005738
(87) Internationale Veröffentlichungsnummer: WO 1999/012949

(56) Entgegenhaltungen:
- EP-A- 0 314 294
- EP-A- 0 335 633
- EP-A- 0 739 988
- WO-A-96/00298
- ZHU Q ET AL: "Detection of Salmonella typhi by polymerase chain reaction" JOURNAL OF APPLIED BACTERIOLOGY, Bd. 80, 1966, Seiten 244-51, XP002096352
- KUR J ET AL: "Identification of Pseudomonas aeruginosa on the basis of polymerase chain reaction amplified DNA spacer polymorphism" ACTA MICROBIOLOGICA POLONICA, Bd. 44, Nr. 2, 1995, Seiten 111-7, XP002096353
- Toschka HY. et al., Nucleic Acid Research (1987), Vol. 15, Nb. 17, page 7182

## Beschreibung

Die Erfindung betrifft Nucleinsäuremoleküle für *Pseudomonas-*Nachweis, einen Kit sowie deren Verwendungen.

### Allgemeiner Hintergrund der Erfindung

Das gram-negative Bakterium *Pseudomonas aeruginosa* ist ein weit-verbreitetes, für den Menschen pathogenes Bakterium, das vor allem für Neugeborene und abwehrgeschwächte Menschen ein hohes gesundheitliches Risiko darstellt. Neben seiner hohen klinischen Relevanz, der häufig vorhandenen Antibiotikaresistenzen und der Bildung von Toxinen, insbesondere des hochgiftigen Exotoxins A (Woods, D.E. and Iglewski, B.H., Rev. Infect. Dis. 5, 714-722 (1983), ist *Pseudomonas aeruginosa* einer der bedeutendsten, bakteriellen Verursacher von Lebensmittelvergiftungen. Für den Nachweis von *Pseudomonas aeruginosa* werden mittels konventioneller Verfahren mindestens 4 Tage benötigt. Die Entwicklung schneller Nachweisverfahren von *Pseudomonas aeruginosa* in Lebensmitteln und klinischen Proben ist daher dringend erforderlich.

Für den routinemäßigen Einsatz zur Erfassung einzelner Mikroorganismen sind in den letzten Jahren eine Reihe neuer Methoden entwickelt worden. Hierzu zählen immunologische Verfahren, die auf dem Einsatz polyvalenter oder monoklonaler Antikörper beruhen und Verfahren, bei denen Nucleinsäure-Sonden zum Nachweis mittels Hybridisierung an keimspezifische Nucleinsäuren eingesetzt werden. Als weitere Methoden werden diejenigen Verfahren beschrieben, die auf einer spezifischen Nucleinsäure-Amplifikation basieren, mit oder ohne anschließende Bestätigungsreaktion durch Nucleinsäure-Hybridisierung. Eingesetzte Verfahren zur Amplifikation von Nucleinsäuren sind z.B. die Polymerase-Kettenreaktion (polymerase chain reaction, PCR) [US Patente 4,683,195; 4,683,202; und 4,965,188], die Ligase-Kettenreaktion [WO Veröffentlichung 89/09835], die "self-sustained sequence replication" [EP 329,822], das "transcription based amplification system" [EP 310,229] und das Qβ RNA-Replikase-System [US Patent 4,957,858].

Die genannten Verfahren auf Nucleinsäure-Basis sind so sensitiv, daß, anders als bei konventionellen mikrobiologischen Verfahren, eine langwierige Anreicherung des nachzuweisenden Mikroorganismus aus der zu untersuchenden Probe entfällt oder stark verkürzt werden kann. Eine Untersuchung auf An- oder Abwesenheit des jeweiligen Mikroorganismus ist daher bei Anwendung der genannten Verfahren auf Nucleinsäure-Basis in der Regel innerhalb eines Tages abgeschlossen. Insbesondere wenn für den Nachweis mittels konventioneller Verfahren mehrere Tage bis Wochen benötigt werden, wird hierdurch eine erhebliche Zeitverkürzung erreicht.

Es sind verschiedene Verfahren auf PCR-Basis zum Nachweis von *Pseudomonas aeruginosa* beschrieben. Mittels Amplifikation einer 369 bp langen DNA-Region aus dem Exotoxin A-Gen konnte selektiv die Anwesenheit von Stämmen der Spezies *Pseudomonas aeruginosa* nachgewiesen werden [Khan et al. (1994), Appl. Environ. Microbiol. 60, 3739-3745]. Zwar wurden mit diesem PCR-System keine Bakterien anderer Spezies erfaßt, jedoch konnte nur bei 96% der insgesamt 130 getesteten *Pseudomonas aeruginosa*-Stämme ein Amplifikat beobachtet werden. Dieses PCR-System ist somit nur bedingt für die Etablierung eines Schnellverfahrens geeignet, mit dem die Anwesenheit aller Stämme von *Pseudomonas aeruginosa* zuverlässig nachgewiesen werden kann.

Mit Hilfe eines weiteren, kürzlich veröffentlichten, auf einer Multiplex-PCR basierenden Verfahrens gelang der selektive Nachweis von fluoreszierenden Pseudomonaden einerseits und *Pseudomonas aeruginosa* andererseits [De Vos et al (1997), J. Clin. Microbiol. 35, 1295-1299]. Jedes der insgesamt 150 getesteten Isolate von *Pseudomonas aeruginosa* konnte mit diesem Verfahren erfaßt werden. Nachteilig ist jedoch, daß das für die selektive Erfassung von *Pseudomonas aeruginosa* herangezogene *oprL-Gen* auch in anderen Spezies der Gattung *Pseudomonas* hochkonserviert ist. So sind die Aminosäuren, die im Bereich der Bindungsstellen der von Voss et al. verwendeten Primer kodiert werden, in *Pseudomonas putida* und *Pseudomonas aeruginosa* identisch. Der Nachweis von *Pseudomonas aeruginosa* beruht somit lediglich auf einigen wenigen unterschiedlichen Basenpaaren bedingt durch die Variation der dritten Position einzelner Aminosäurecodons. Dies birgt erfahrungsgemäß eine große Gefahr des Auftretens von falsch-positiven bzw. falsch negativen Ergebnissen.

Das beschriebene Multiplex-PCR-System bietet zudem wegen der hohen Konservierung der *oprI* und *oprL*-Gene wohl kaum die Möglichkeit, durch z.B. Einsatz verschiedener Sonden im Anschluß an die PCR-Reaktion, auch andere klinisch relevante Spezies der Gattung *Pseudomonas* nachzuweisen, wie z.B. *Pseudomonas fluorescens, Pseudomonas mendocina, Pseudomonas putida* oder *Pseudomonas stutzeri*.

Ziel der hier dargestellten Erfindung war die Etablierung von Nucleinsäuresequenzen, deren Einsatz als Primer und/oder Sonden eine möglichst vollständige Erfassung aller Vertreter der Spezies *Pseudomonas aeruginosa* sicherstellt. Ein weiteres Ziel der Erfindung war das Auffinden eines Genom-Bereiches, der innerhalb verschiedener Spezies der Gattung *Pseudomonas* ausreichend hohe Sequenz-Variabilität aufweist, um optional auch den Nachweis anderer Spezies der Gattung *Pseudomonas* zu ermöglichen, z.B. durch.Einsatz verschiedener Varianten von Primern und/oder Sonden in der PCR bzw. im Anschluß an die PCR.

Je nach Größe der zu detektierenden Gruppe von Mikroorganismen und evolutionäre Verwandtschaft (Ähnlichkeit) von abzugrenzenden (nicht zu erfassenden) Mikroorganismen erfordert ein auf differentiellen DNA-Sequenzen basierender Nachweis sehr umfangreiche Vorarbeiten, um jeweils geeignete DNA-Sequenzen mit der gewünschten Spezifität zu finden. Die hier dargelegte Erfindung betrifft solche DNA-Sequenzen, mit denen der Schnellnachweis von Bakterien der Gattung *Pseudomonas*, insbesondere von *Pseudomonas aeruginosa* möglich ist.

Ferner sind Nucleinsäuremoleküle bekannt, welche als Sonden oder Primer für den Nachweis von Mikroorganismen eingesetzt werden können, speziell für die Detektion von *Pseudomonas aeruginosa* (WO 96/00298 und Acta Microbiologica Polonica, 44 (1995) 111-117), wobei die Nucleinsäuremoleküle aus der 16S-23S-intergenischen Region gewonnen werden. *Pseudomonas aeruginosa* kann dabei von anderen *Pseudomonas* s*peciis* als auch von Bakterien anderer Genera unterschieden werden.

Auch ist es bekannt, daß die 23S-5S-intergenische Region für Bakterien, bei denen es sich nicht um *Pseudomonas-speciis* handelt, erfolgreich zur Isolierung von speciis- und generaspezifischen Nucleinsäuremolekülen verwendet werden kann (J. Applied Bakteriology, 80 (1996) 244-251 und EP 0 739 988).

### Beschreibung der Erfindung

Gemäß einer Ausführungsform wird die der Offenbarung zugrundeliegene Aufgabe durch ein Nucleinsäuremolekül gelöst, das dadurch gewinnbar ist, daß man von mehreren Stämmen ausgeht, die einerseits einer nachzuweisenden Gruppe von Bakterien des Genus *Pseudomonas* und andererseits nicht-nachzuweisenden Bakterien angehören,
(a) in an sich bekannter Weise aus einem *Pseudomonas*-Stamm dieser Gruppen (erster Stamm) genomische DNA isoliert,
(b) in an sich bekannter Weise die 23S/5S-intergenische Region gegebenenfalls mit dem direkt angrenzenden 23S-Bereich und/oder dem direkt angrenzenden 5S-Bereich amplifiziert und das Amplifikationsprodukt gewinnt (erstes Amplifikationsprodukt),
(c) mit einem zweiten, dritten,..... und/oder nten *Pseudomonas-*Stamm dieser Gruppen jeweils gemäß Stufen (a) und (b) genomische DNA isoliert, die 23S/5S- intergenische Region gegebenenfalls mit dem direkt angrenzenden 23S-Bereich und/oder dem direkt angrenzenden 5S-Bereich amplifiziert und das Amplifikationsprodukt gewinnt (zweites, drittes, ..... ntes Amplifikationsprodukt),
(d) in an sich bekannter Weise die DNA-Sequenz von gemäß (b) und (c) gewonnenen Amplifikationsprodukten bestimmt und die DNA-Sequenz des Amplifikationsproduktes gemäß (b) mit der DNA-Sequenz eines oder mehrerer Amplifikationsprodukte gemäß (c) vergleicht und
(e) als Primer oder als Sonde ein Nucleinsäuremolekül in an sich bekannter Weise gewinnt, mit dessen Hilfe sich die nachzuweisende Gruppe von Bakterien des Genus *Pseudomonas* von der nicht-nach-zuweisenden Gruppe von Bakterien des Genus *Pseudomonas* anhand von Unterschieden an mindestens einer Nukleotidposition im Sequenzbereich des Nucleinsäuremoleküls unterscheiden läßt.

Das Nucleinsäuremolekül kann dadurch gewinnbar sein, daß man von Stämmen ausgeht, die einerseits nachzuweisenden Bakterien des Genus *Pseudomonas* und andererseits nicht nachzuweisenden Bakterien eines anderen Genus (anderer Genera) als *Pseudomonas* angehören.

Gemäß einer weiteren Ausführungsform wird die der Offenbarung zugrundeliegende Aufgabe durch ein Nucleinsäuremolekül gelöst, das dadurch gewinnbar ist, daß man von mehreren Stämmen ausgeht, die einer nachzuweisenden und einer nicht-nachzuweisenden Gruppe von Bakterien des Genus *Pseudomonas* angehören,
(a) in an sich bekannter Weise aus einem *Pseudomonas*-Stamm dieser Gruppen (erster Stamm) genomische DNA isoliert,
(b) in an sich bekannter Weise die 23S/5S-intergenische Region gegebenenfalls mit dem direkt angrenzenden 23S-Bereich und/oder dem direkt angrenzenden 5S-Bereich amplifiziert und das Amplifikationsprodukt gewinnt (erstes Amplifikationsprodukt),
(c) mit einem zweiten, dritten,..... und/oder nten *Pseudomonas-*Stamm dieser Gruppen jeweils gemäß Stufen (a) und (b) genomische DNA isoliert, die 23S/5S- intergenische Region mit dem direkt angrenzenden 23S-Bereich und/oder dem direkt angrenzenden 5S-Bereich amplifiziert und das Amplifikationsprodukt gewinnt (zweites, drittes, ...... ntes Amplifikationsprodukt),
(d) in an sich bekannter Weise die DNA-Sequenz von gemäß (b) und (c) gewonnenen Amplifikationsprodukten bestimmt und die DNA-Sequenz des Amplifikationsproduktes gemäß (b) mit der DNA-Sequenz eines oder mehrerer Amplifikationsprodukte gemäß (c) vergleicht und
(e) als Primer oder als Sonde ein Nucleinsäuremolekül in an sich bekannter Weise gewinnt, mit dessen Hilfe sich die nachzuweisende Gruppe von Bakterien des Genus *Pseudomonas* von der nicht-nach-zuweisenden Gruppe von Bakterien des Genus *Pseudomonas* anhand von Unterschieden an mindestens einer Nukleotidposition im Sequenzbereich des Nucleinsäuremoleküls unterscheiden läßt.

Das Nucleinsäuremolekül kann dadurch gewinnbar sein, daß man von Stämmen ausgeht, die einer nachzuweisenden Gruppe von Bakterien der Species *Pseudomonas aeruginosa* und einer nicht-nachzuweisenden Gruppe von Bakterien anderer Species angehören.

Ferner betrifft die Offenbarung ein Nucleinsäuremolekül der SEQ ID NO 1 oder der hierzu komplementären Sequenz.

Ferner betrifft die Offenbarung ein derartiges Nucleinsäuremolekül mit einer gegenüber dem vorstehenden Nucleinsäuremolekül verkürzten Sequenz, und zwar der Sequenz des Bereiches oder im Bereich der Nukleotidpositionen 12 bis 131. Die erfindungsemäßen Nukleinsäuremolekül sind in Anspruch 1 definiert.

Ferner betrifft die Offenbarung ein derartiges Nucleinsäuremolekül mit einer gegenüber einem Nucleinsäuremolekül der SEQ ID NO 1 verkürzten Sequenz, nämlich
(i) der SEQ ID NO 3 oder
(ii) der SEQ ID NO 4 oder
(iii) der SEQ ID NO 5 oder
(iv) der zu (i), (ii) und (iii) jeweils komplementären Sequenz.

Ferner betrifft die Offenbarung ein Nucleinsäuremolekül der SEQ ID NO 2 oder der hierzu komplementären Sequenz.

Ein Nucleinsäuremolekül kann dadurch gekennzeichnet sein, daß es hinsichtlich seiner Sequenz bei mindestens 10 aufeinanderfolgenden Nukleotiden seiner Nukleotidkette
(i) mit einem Nucleinsäuremolekül gemäß einem der vorhergehenden Ansprüche identisch ist oder
(ii) in 9 von 10 aufeinanderfolgenden Nukleotiden mit einem Nucleinsäuremolekül gemäß einem der vorhergehenden Ansprüche übereinstimmt oder
(iii) in 8 von 10 aufeinanderfolgenden Nukleotiden mit einem Nucleinsäuremolekül gemäß einem der vorhergehenden Ansprüche übereinstimmt oder
(iv) zu mindestens 90 % mit einem Nucleinsäuremolekül gemäß einem der vorhergehenden Ansprüche homolog ist.

Ein derartiges Nucleinsäuremolekül kann dadurch gekennzeichnet sein, daß es 10 bis 250 und vorzugsweise 15 bis 30 Nukleotide lang ist.

Ein erfindungsgemäßes Nucleinsäuremolekül kann dadurch gekennzeichnet sein, daß es einzelsträngig oder doppelsträngig vorliegt.

Ein erfindungsgemäßes Nucleinsäuremolekül kann dadurch gekennzeichnet sein, daß es
(i) als DNA oder
(ii) als (i) entsprechende RNA oder
(iii) als PNA vorliegt.
Das Nucleinsäuremolekül kann gegebenenfalls in einer für analytische Nachweisverfahren, insbesondere auf Basis von Hybridisierung und/oder Amplifizierung, an sich bekannten Weise modifiziert ist.

So kann ein Nucleinsäuremolekül dadurch modifiziert sein, daß bis zu 20 % der Nukleotide von mindestens 10 aufeinanderfolgenden Nukleotiden seiner Nukleotidkette, insbesondere 1 oder 2 Nukleotide, durch für Sonden und/oder Primer an sich bekannte analoge Bausteine ersetzt sind, insbesondere durch Nukleotide, die bei Bakterien nicht natürlich vorkommen.

Ferner kann das erfindungsgemäße Nucleinsäuremolekül dadurch oder zusätzlich dadurch modifiziert bzw. markiert sein, daß es in einer für analytische Nachweisverfahren an sich bekannten Weise eine oder mehrere radioaktive Gruppen, farbige Gruppen, fluoreszierende Gruppen, Gruppen zur Immobilisierung an fester Phase und/oder Gruppen für eine indirekte oder direkte Reaktion, insbesondere eine enzymatische Reaktion, insbesondere mit Hilfe von Antikörpern, Antigenen, Enzymen und/oder Substanzen mit Affinität zu Enzymen oder Enzymkomplexen, und/oder anderweitig modifizierende oder modifizierte Gruppen nucleinsäureähnlichen Aufbaus aufweist.

Gemäß einer weiteren Ausführungsform wird die der Erfindung zugrundeliegende Aufgabe durch einen Kit für analytische Nachweisverfahren gelöst, insbesondere zum Nachweis von Bakterien der Gattung *Pseudomonas*, wobei der Kit gekennzeichnet ist durch ein oder mehrere erfindungsgemäße Nucleinsäuremoleküle gemäß Anspruch 1.

Gemäß einer weiteren Ausführungsform wird die der Erfindung zugrundeliegende Aufgabe durch eine Verwendung von einem oder mehreren erfindungsgemäßen Nucleinsäuremolekülen gemäß Anspruch 1 oder eines erfindungsgemäßen Kits zum Nachweis der An- oder Abwesenheit von Bakterien gelöst, welche einer Gruppe von Bakterien der Gattung *Pseudomonas* angehören.

Die erfindungsgemäße Verwendung kann dadurch gekennzeichnet sein, daß die Gruppe von Bakterien der Gattung *Pseudomonas* verschiedene Stämme von *Pseudomonas aeruginosa* umfaßt oder durch diese Stämme gebildet wird.

Eine derartige erfindungsgemäße Verwendung kann dadurch gekennzeichnet sein, daß es sich bei der Gruppe von Bakterien der Gattung *Pseudomonas* ausschließlich um *Pseudomonas aeruginosa*-Stämme handelt.

Ferner kann eine erfindungsgemäße Verwendung dadurch gekennzeichnet sein, daß man eine Nucleinsäurehybridisierung und/oder eine Nucleinsäureamplifikation durchführt.

Ferner kann eine erfindungsgemäße Verwendung dadurch gekennzeichnet sein, daß man eine Polymerase-Kettenreaktion als Nucleinsäureamplifikation durchführt.

Ferner kann eine erfindungsgemäße Verwendung dadurch gekennzeichnet sein, daß man den Nachweis dadurch durchführt, daß man die nachzuweisenden Bakterien von nicht-nachzuweisenden Bakterien anhand von Unterschieden der genomischen DNA und/oder RNA an mindestens einer Nukleotidposition im Bereich eines erfindungsgemäßen Nucleinsäuremoleküls unterscheidet.

Ferner kann eine Verwendung dadurch gekennzeichnet sein, daß man anhand von Unterschieden im Bereich eines Nucleinsäuremoleküls der SEQ ID NO 1 oder der hierzu komplementären Sequenz unterscheidet.

Zur Detektion von spezifischen Mikroorganismen mittels Nucleinsäure-Hybridisierung oder -Amplifikation werden erfindungsgemäß also keimspezifische Oligonukleotide gemäß Anspruch 1 eingesetzt. **Keimspezifische Oligonukleotide** sind Nucleinsäuren, 10 bis 250 Basen (vorzugsweise 15 bis 30 Basen) lang, deren Basensequenz charakteristisch für einen spezifischen Mikroorganismus oder eine Gruppe von Mikroorganismen ist. Eine Hybridisierung an DNA bzw. eine Amplifikation von DNA bei Einsatz dieser keimspezifischen Oligonukleotide (z.B. als Primer oder Sonden) mit den oben genannten Verfahren kann, unter geeigneten Reaktionsbedingungen, nur dann erfolgen, wenn die DNA der jeweils nachzuweisenden Mikroorganismen anwesend ist.

Prokaryontische Ribosomen beinhalten drei distinkte Nucleinsäurekomponenten, welche allgemein als 5S, 16S und 23S rRNA (ribosomale Ribonucleinsäure) bekannt sind. Die genetische Information für diese Ribonucleinsäuren (rDNA) ist im Genom typischerweise in Form von Tandems angeordnet. Die Organisation einer solchen Einheit ist 16S-23S-5S, wobei die drei Gene durch kurze hypervariable intergenische Regionen voneinander getrennt sind. Die Einheiten sind im Genom mehrfach vorhanden, wobei die Anzahl der sich wiederholenden Einheiten in verschiedenen Bakterien variieren kann. Die hohe Konservierung der DNA-Sequenz im Bereich der 16S rDNA, der 23S rDNA und der 5S rDNA über das gesamte Bakterienreich ermöglicht ein Design von **nicht-spezifischen Oligonukleotiden** auch ohne genaue Kenntnis der DNA-Sequenzen der zu untersuchenden Mikroorganismen. Solche nicht-spezifischen Oligonukleotide sind charakteristisch für eine größere, in der Regel phylogenetisch verwandte Gruppe von Mikroorganismen. Durch Einsatz dieser nicht-spezifischen Oligonukleotide gelingt dem Fachmann, z.B. nach entsprechenden Vorversuchen durch DNA-Amplifikation mittels PCR, die Isolation von rDNA-Fragmenten, z.B. der 23S/5S intergenischen Region eines beliebigen Mikroorganismus. Durch DNA-Sequenzierung kann dann die Sequenz der hypervariablen intergenischen Regionen des betreffenden Mikroorganismus bestimmt werden.

DNA-Sequenzierung der 23S/5S intergenischen Region einer möglichst großen Anzahl **nachzuweisender** Bakterien (z.B von verschiedenen Pseudomonas-Spezies) einerseits und nachfolgender Vergleich dieser DNA-Sequenzen andererseits erlauben das Auffinden von DNA-Bereichen, die in der untersuchten Gruppe (z.B. alle Pseudomonas-Spezies) nicht oder nur unwesentlich verändert ist.

DNA-Sequenzierung der 23S/5S intergenischen Region ausgewählter **nicht-nachzuweisender** Bakterien (z.B. Bakterien die nicht zur Gattung *Pseudomonas* gehören) einerseits und nachfolgender Vergleich dieser DNA-Sequenzen mit der Sequenz der nachzuweisenden Bakterien (z.B. verschiedener *Pseudomonas-*Spezies) andererseits erlaubt das Auffinden von DNA-Sequenzen, die für die nachzuweisenden Bakterien (z.B. alle *Pseudomonas-*Spezies) charakteristisch sind. Aus diesen DNA-Sequenzen können wiederum Oligonukleotide abgeleitetet werden, die als Primer und/oder Sonden in auf Nucleinsäuren basierenden Verfahren einsetzbar sind, mit dem Ziel, die jeweilige Gruppe von Bakterien (z.B. alle Spezies der Gattung *Pseudomonas*) spezifisch nachzuweisen.

Die in der vorliegenden Erfindung beschriebenen DNA-Sequenzen zum Nachweis von Bakterien der Gattung *Pseudomonas*, insbesondere Bakterien der Spezies *Pseudomonas aeruginosa*, basieren auf der 23S/5S intergenischen Region und dem direkt angrenzenden Bereich der 23S rDNA. Die DNA-Sequenz in dieser Region wurde für eine Vielzahl von Bakterien bestimmt. Nach exakten Sequenzvergleichen wurden keimspezifische Nucleinsäuresequenzen bestimmt, für Primer und/oder Sonden für einen Einsatz in einem Spezies-/Genus-spezifischen Nachweisverfahren benutzt werden können.

Zum Nachweis der jeweiligen Gruppe von Mikroorganismen werden Nucleinsäuren, vorzugsweise genomische DNA, zunächst aus den in einer zu untersuchenden Probe bzw. Bakterienkultur enthaltenen Zellen freigesetzt. Mittels Nucleinsäure-hybridisierung kann dann, und zwar unter Einsatz der erfindungsgemäßen keimspezifischen Oligonukleotide als Sonde, der **direkte** Nachweis von keimspezifischen Nucleinsäuresequenzen in der zu untersuchenden Probe erfolgen. Geeignet hierzu sind verschiedene dem Fachmann bekannte Verfahren, wie z.B. "Southern blot" oder "dot blot".

Bevorzugt ist jedoch, vor allem wegen der höheren Empfindlichkeit, ein indirektes Nachweisverfahren, bei dem die gesuchten DNA/RNA-Sequenzen zunächst mittels der o.g. Verfahren zur Amplifikation von Nucleinsäuren, vorzugsweise PCR, amplifiziert werden.

Die Amplifikation von DNA/RNA unter Verwendung der genannten Verfahren kann unter Einsatz von keimspezifischen Oligonukleotiden als Primer erfolgen. Dabei werden nur in dem Fall spezifische Amplifikate gebildet, in dem DNA/RNA des nachzuweisenden Mikroorganismus anwesend ist. Durch eine nachgeschaltete Detektionsreaktion unter Verwendung von keimspezifischen Oligonukleotiden als Sonden kann die Spezifität des Nachweisverfahrens erhöht werden. Für diese nachgeschaltete Detektionsreaktion ist ebenso die Verwendung von nicht-keimspezifischen Oligonukleotiden möglich.

Alternativ kann die Nucleinsäure-Amplifikation auch in Anwesenheit eines oder mehrerer nicht-spezifischer Oligonukleotide durchgeführt werden, so daß möglicherweise auch DNA/RNA anderer, nicht-nachzuweisender Mikroorganismen amplifiziert werden kann. Ein derartiges Amplifikationsverfahren ist in der Regel weniger spezifisch und sollte daher durch eine nachfolgende Detektionsreaktion mit einem oder mehreren keimspezifischen Oligonukleotid(en) als Sonde(n) abgesichert werden.

Dem Fachmann sind verschiedene Verfahren bekannt, mit denen die bei den indirekten Verfahren entstehenden Amplifikationsprodukte nachgewiesen werden können. Dazu gehören u.a. die Visualisierung mittels Gelelektrophorese, die Hybridisierung von Sonden an immobilisierte Reaktionsprodukte [gekoppelt an Nylon- oder Nitrocellulose-Filter ("Southern blots") oder z.B. an "beads" oder Mikrotiterplatten] und die Hybridisierung der Reaktionsprodukte an immobilisierte Sonden (z.B. "reverse dot blots" oder mit Sonden gekoppelte "beads" oder Mikrotiterplatten).

Es sind eine Vielzahl verschiedener Varianten beschrieben, mit denen keimspezifische Oligonukleotide (z.B. Sonden und Primer) für die beschriebenen direkten oder indirekten Nachweisverfahren markiert bzw. modifiziert werden können. So können diese beispielsweise radioaktive, farbige, fluoreszierende oder anderweitig modifizierte bzw. modifizierende Gruppen enthalten, beispielsweise Antikörper, Antigene, Enzyme bzw. andere Substanzen mit Affinität zu Enzymen oder Enzymkomplexen. Sonden bzw. Primer können entweder natürlich vorkommende oder synthetisch hergestellte doppelsträngige oder einzelsträngige DNA oder RNA sein bzw. modifizierte Formen von DNA oder RNA, wie z.B. PNA (bei diesen Molekülen sind die Zucker-Einheiten durch Aminosäuren oder Peptide ausgetauscht). Einzelne oder mehrere Nukleotide der Sonden oder Primer können gegen analoge Bausteine (wie z.B. Nukleotide, die in der Ziel-Nucleinsäure nicht natürlich vorkommen) ersetzt sein. Bei den o.g. indirekten Nachweisverfahren kann Detektion auch über ein internmarkiertes Amplifikat geführt werden. Dies kann z.B. über den Einbau von modifizierten (z.B. an Digoxigenin oder an Fluorescein gekoppelten) Nukleosidtriphosphaten während der Amplifikationsreaktion erfolgen.

Geeignet als keimspezifische Oligonukleotide sind Nucleinsäuren, vorzugsweise 10 bis 250 Basen und insbesondere 15 bis 30 Basen lang, die mindestens in einer 10 Basen langen Sequenz mit den unten angegebenen Sequenzen 1 bis 5 oder den hierzu komplementären Sequenzen übereinstimmen. Geringere Abweichungen (1 bis 2 Basen) in dieser 10 Basen langen Sequenz sind möglich, ohne daß die jeweils angegebene Spezifität bei der Amplifikation und/oder Hybridisierung verloren geht. Dem Fachmann ist bekannt, daß im Falle solcher geringeren Abweichungen die Reaktionsbedingungen entsprechend verändert werden müssen; vgl. beispielsweise T. Maniatis, Molecular Cloning, Herausgeber G. Sambrook &. E.F. Fritsch, Cold Spring Harbour Laboratory Press, 1989.

Die Sequenz von *Pseudomonas aeruginosa* (ATCC 10145) im Bereich der 23S/5S intergenischen Region lautet:

(Sequenz 1 = SEQ ID NO 1))
ATAACACCCAAACAATCTGAYGATTGTGTTGTAAGGTGAAGTCGACGAACCGAAAGTTCG
CATGAACCGCAAACACCTTGAAATCACATACCTGAATCCGGATAGACGTAAGCCCAAGCGAA
CGGATAT

Außerdem wurde die Sequenz im Bereich der 23S/5S-intergenischen Region für 6 weitere Stämme der Spezies *Pseudomonas aeruginosa* sowie für mindestens je einen Stamm der folgenden Spezies bestimmt: *Pseudomonas asplenii, Pseudomonas citronellosis, Pseudomonas corrugata, Pseudomonas fluorescens, Pseudomonas fragi, Pseudomonas mendocina, Pseudomonas pseudoalcaligenes, Pseudomonas putida, Pseudomonas stutzeri, Pseudomonas syringae*. Die Sequenzvergleiche ergaben, daß sich mehrere, von Sequenz 1 abgeleitete Oligonukleotide für den selektiven Nachweis von Bakterien der Spezies *Pseudomonas aeruginosa* eignen. Geeignet für solche keimspezifischen Oligonukleotide ist die Sequenz der Region (12-131).

Von Sequenz 1 wurden folgende, als Primer für die PCR (Sequenz 3 und 5) und als Sonde (Sequenz 4), besonders geeignete Oligonukleotide abgeleitet.

Oligonukleotid Pa1 (Sequenz 2) entspricht Position 2823-2842 eines 23S rRNA-Gens von *Pseudomonas aeruginosa* ATCC 10145 [Toschka et al. (1987), Nucleic Acids. Res. 15, 7182]:
Oligonukleotid Pa1: (Sequenz 2 = SEQ ID NO 2) 5'-GATAGGCTGGGTGTGTAAGC-3'
Oligonukleotid Pa2: (Sequenz 3 = SEQ ID NO 3) 5'-CTTGGGCTTACGTCTATCCG-3'
Oligonukleotid Pa3: (Sequenz 4 = SEQ ID NO 4) 5'-TTCAGGTATG TGATTTCAAG GTG-3'
Oligonukleotid Pa4: (Sequenz 5 = SEQ ID NO 5) 5'-GACGATTGTGTGTTGTAAGGTGA

### Beispiel 1: Nachweis von Bakterien der Spezies Pseudomonas aeruginosa mit der Polymerase-Kettenreaktion

Aus Reinkulturen der in Tabelle 1 aufgeführten Bakterien wurde DNA mittels Standardverfahren isoliert. Je ca. 10 bis 100 ng dieser DNA-Präparationen wurde dann in Gegenwart von je 0,4 µM Oligonukleotid Pa1 und Pa2 oder Pa4 und Pa2, 200 µM dNTP's (Boehringer Mannheim), 4 mM MgCl₂, 16 mM (NH₄)₂SO₄, 67 mM Tris/HCl (pH 8,8), 0,01 % Tween 20 und 0,03 U/µl Taq-Polymerase (Biomaster) in die PCR eingesetzt. Die PCR wurde in einem Perkin-Elmer 9600 (Pa1 und Pa2)/ Biometra TRIO-Thermoblock (Pa4 und Pa2) Thermocycler mit den nachfolgend aufgeführten Thermoprofilen durchgeführt:
a) Amplifikation mit Oligonukleotid Pa1 und Pa2

| | | |
|---|---|---|
| - initiale Denaturierung | 95 °C | 5 min |
| | | |
| - 1. Amplifikation (15 Zyklen) | 94 °C | 35 sek |
| | 68 °C | 30 sek |
| | 72 °C | 30 sek |
| | | |
| - 2. Amplifikation (20 Zyklen) | 99 °C | 35 sek |
| | 64 °C | 30 sek |
| | 72 °C | 30 sek |
| | | |
| - finale Synthese | 72 °C | 5 min |

b) Amplifikation mit Oligonukleotid Pa4 und Pa2

| | | |
|---|---|---|
| - initiale Denaturierung | 95 °C | 5 min |
| | | |
| - Amplifikation (35 Zyklen) | 95 °C | 30 sek |
| | 62 °C | 30 sek |
| | 72 °C | 20 sek |
| | | |
| - finale Synthese | 72 °C | 5 min |

Nach Beendigung der PCR-Reaktion wurden die Amplifikationsprodukte mittels Agarose-Gelelektrophorese aufgetrennt und durch Anfärbung mit Ethidiumbromid visualisiert. Die erwarteten Produkte von 191 bp /102 bp Länge wurden nur in den Fällen beobachtetet, in denen DNA von Stämmen der Spezies *Pseudomonas aeruginosa* anwesend war (vergleiche Tabelle 1), nicht aber bei Anwesenheit von DNA der anderen getesteten Bakterien. Nach Beendigung des Laufes wurde die in den Gelen enthaltene DNA mittels Standard-Methoden auf Nylon-Filter transferiert und zur Überprüfung der Spezifität mit dem am 5'-Ende biotinylierten Oligonukleotid Pa3 (Sequenz 4) hybridisiert. Die Hybridisierung erfolgte in 5 x SSC , 2 % Blocking Reagenz, 0,1 % Laurylsarcosin, 0,02 % SDS und 5 pmol/ml Sonde für 4 h bei 48°C. Gewaschen wurde in 2 x SSC, 0,1 % SDS für 2 x 5 min bei Raumtemperatur und in 2 x SSC, 0,1 % SDS für 1 x 15 min bei 48°C. Die Detektion erfolgte nach Standard-Methoden mittels Alkalischer Phosphatase-Konjugate (Extravidin, Fa SIGMA, # E-2636) in Anwesenheit von 5-Bromo-4-chloro-3-indolylphosphat und 4-Nitro-Blue Tetrazoliumchlorid (Fa. Boehringer Mannheim).

Auf den Filtern wurde nur in den Fällen eine Bande beobachtet, in denen zuvor auch eine Bande auf dem Agarose-Gel sichtbar war (siehe Tabelle 1). Somit wurde mittels PCR und mittels Hybridisierung die Anwesenheit sämtlicher der 86 getesteten *Pseudomonas* aeruginosa-Stämme nachgewiesen. Hingegen wurde keiner der getesteten nicht zu dieser Spezies gehörenden Bakterienstämme mit diesem System erfaßt.

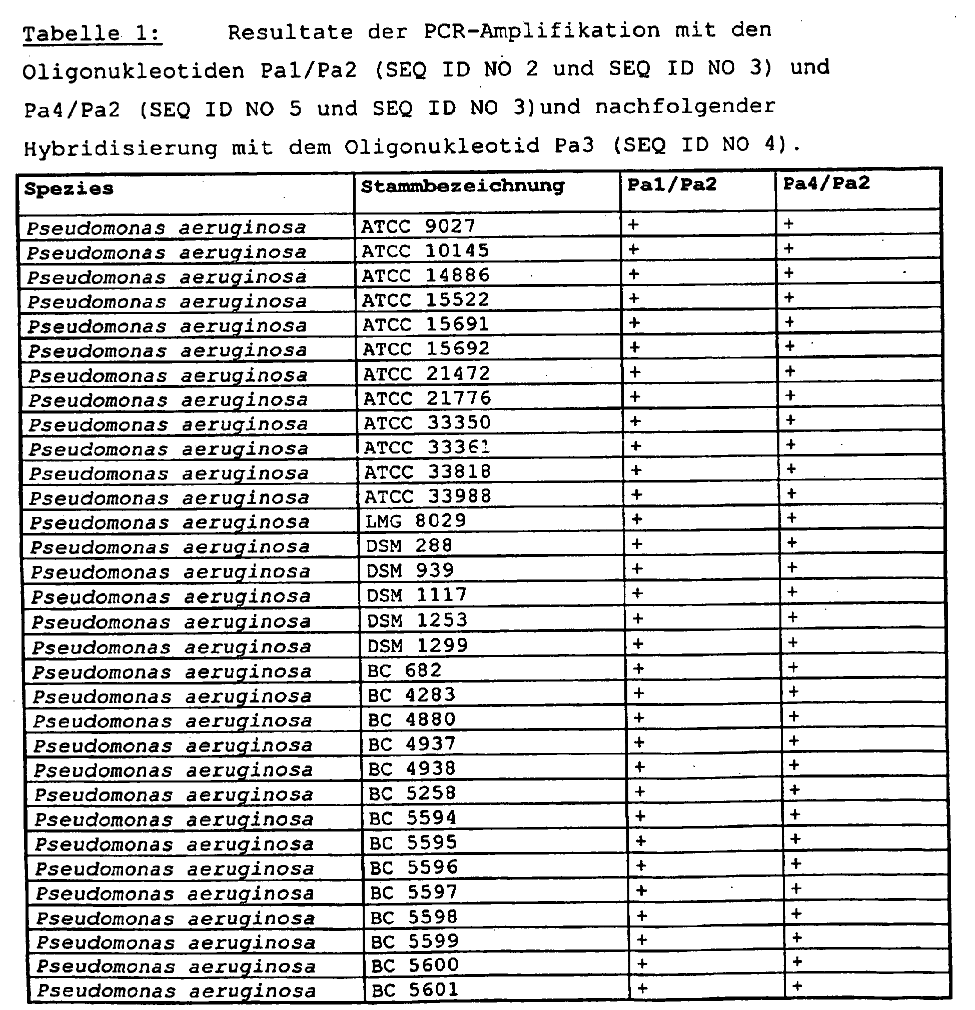

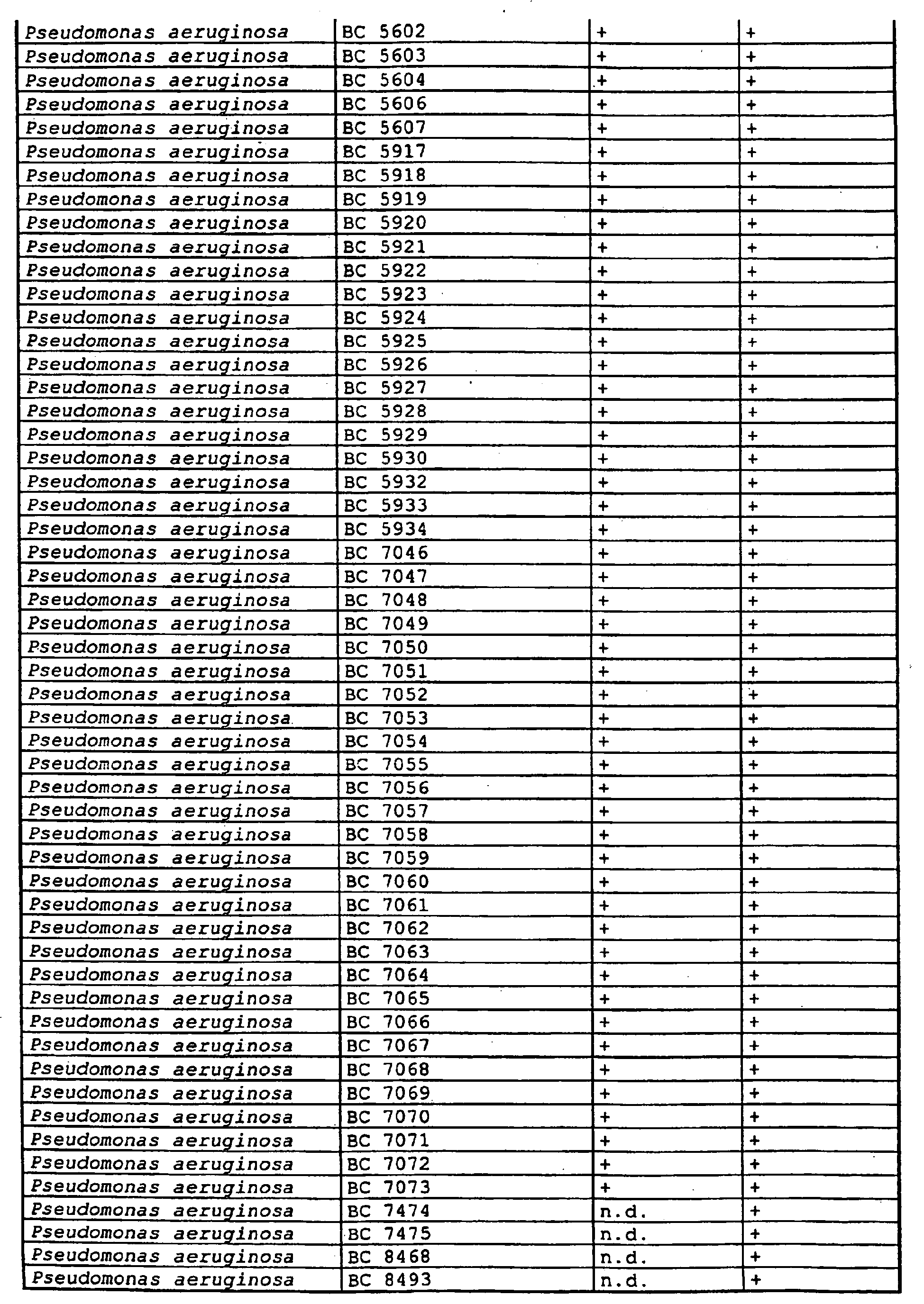

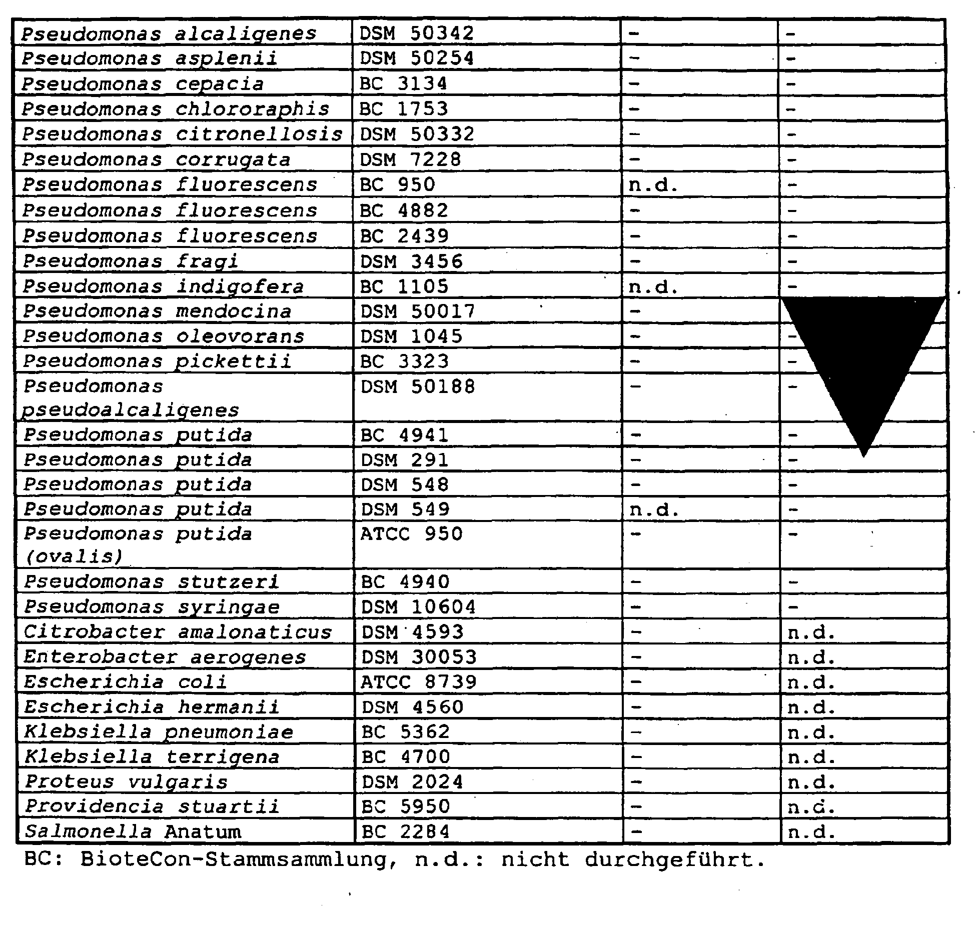

## Patentansprüche

1. Keimspezifisches Oligonucleotid zum Nachweis von Pseudomonas aeruginosa-Stämmen, **dadurch gekennzeichnet, dass** es
10 bis 30 Nucleotide lang ist und mindestens 90 % Identität zu SEQ ID NO 1 im Bereich der Nucleotidpositionen 12 bis 131; SEQ ID NO 3; SEQ ID NO 4; SEQ ID NO 5; oder der jeweils komplementären Sequenz aufweist.

2. Keimspezifisches Oligonucleotid nach Anspruch 1, **dadurch gekennzeichnet, dass** das Oligonucleotid die SEQ ID NO 1 im Bereich der Nucleotidpositionen 12 bis 131; die SEQ ID NO 3; die SEQ ID NO 4; die SEQ ID NO 5; oder die jeweils komplementäre Sequenz ist.

3. Keimspezifisches Oligonucleotid nach einem der vorhergehenden Ansprüche, **dadurch gekennzeichnet, dass** es einzelsträngig oder doppelsträngig vorliegt.

4. Keimspezifisches Oligonucleotid nach einem der vorhergehenden Ansprüche, **dadurch gekennzeichnet, dass** es als
(i) DNA oder
(ii) als (i) entsprechende RNA oder
(iii) als PNA vorliegt.

5. Keimspezifisches Oligonucleotid nach Anspruch 3 bis 4, **dadurch gekennzeichnet, dass** das keimspezifisches Oligonucleotid **dadurch** oder zusätzlich **dadurch** modifiziert ist, dass es in einer für analytische Nachweisverfahren an sich bekannten Weise als Marker eine oder mehrere radioaktive Gruppen, farbige Gruppen, fluoreszierende Gruppen, Gruppen zur Immobilisierung an fester Phase und/oder Gruppen für eine indirekte oder direkte Reaktion und/oder anderweitig modifizierende oder modifizierte Gruppen nucleinsäureähnlichen Aufbaus aufweist.

6. Keimspezifisches Oligonucleotid nach Anspruch 5, **dadurch gekennzeichnet, dass** es Gruppen für eine indirekte oder direkte enzymatische Reaktion aufweist.

7. Keimspezifisches Oligonucleotid nach Anspruch 5 oder 6, **dadurch gekennzeichnet, dass** es Gruppen für eine indirekte oder direkte Reaktion mit Hilfe von Antikörpern, Antigenen, Enzymen und/oder Substanzen mit Affinität zu Enzymen Oder Enzymkomplexen aufweist.

8. Kit zum Nachweis von Pseudomonas aeruginosa, **dadurch gekennzeichnet, dass** er ein oder mehrere keimspezifische Oligonucleotide gemäß einem der vorhergehenden Ansprüche sowie gegebenenfalls ein Oligonucleotid der SEQ ID NO 2 oder Hilfsstoffe enthält.

9. Verwendung ein oder mehrerer keimspezifischer Oligonucleotide gemäß einem der Ansprüche 1 bis 7 oder eines Kits gemäß Anspruch 8 zum Nachweis der An- oder Abwesenheit von Pseudomonas aeruginosa-Stämmen.

10. Verwendung nach Anspruch 9, **dadurch gekennzeichnet, dass** man eine Nucleinsäurehybridisierung und/oder eine Nucleinsäureamplifikation durchführt.

11. Verwendung nach Anspruch 10, **dadurch gekennzeichnet, dass** man eine Polymerase-Kettenreaktion als Nucleinsäureamplifikation durchführt, wobei als Primer SEQ ID NO 2 und SEQ ID NO 3 oder SEQ ID NO 3 und SEQ ID NO 5 eingesetzt werden.

12. Verwendung nach einem der Ansprüche 9 bis 11, **dadurch gekennzeichnet, dass** man die nachzuweisenden Bakterien von nicht-nachzuweisenden Bakterien anhand von Unterschieden der genomischen DNA und/oder RNA an mindestens einer Nucleotidposition im Bereich eines keimspezifischen Oligonucleotids gemäß einem der Ansprüche 1 bis 7 unterscheidet.

13. Verwendung nach Anspruch 12, **dadurch gekennzeichnet, dass** man anhand von Unterschieden im Bereich eines keimspezifischen Oligonucleotids gemäß Anspruch 1 unterscheidet.

## Claims

1. Germ-specific oligonucleotide for detection of *Pseudomonas aeruginosa* strains, **characterised in** having a length of from 10 to 30 nucleotides and an identity of at least 90 % to SEQ ID NO 1 in the region of nucleotide positions 12 to 131; SEQ ID NO 3; SEQ ID NO 4; SEQ ID NO 5; or each complementary sequence thereof.

2. Germ-specific oligonucleotide according to claim 1, **characterized in that** the oligonucleotide is SEQ ID NO 1 in the region of the nucleotide positions 12 to 131; SEQ ID NO 3; SEQ ID NO 4; SEQ ID NO 5; or each complementary sequence thereof.

3. Germ-specific oligonucleotide according to one of the preceding claims, **characterised in that** it is single-stranded or double-stranded.

4. Germ-specific oligonucleotide according to one of the preceding claims, **characterised in that** it is present
(i) as DNA or
(ii) as RNA corresponding to (i) or
(iii) as PNA.

5. Germ-specific oligonucleotide according to claim 3 to 4, **characterised in that** the germ-specific oligonucleotide has been modified or additionally modified in a manner known *per se* as marker in such a manner that it comprises one or more radioactive groups, coloured groups, fluorescent groups, groups for immobilisation on a solid phase and/or groups for an indirect or direct reaction and/or otherwise modifying or modified groups of nucleic-acid-like structure for analytical detection processes.

6. Germ-specific oligonucleotide according to claim 5, **characterised in that** it comprises groups for an indirect or direct enzymatic reaction.

7. Germ-specific oligonucleotide according to one of claims 5 or 6, **characterised in that** it comprises groups for an indirect or direct reaction using antibodies, antigens, enzymes and/or substances having an affinity for enzymes or enzyme complexes.

8. Kit for the detection of *Pseudomonas aeruginosa* **characterised in that** it comprises one or more germ-specific oligonucleotide(s) according to one of the preceding claims and optionally an oligonucleotide of SEQ ID NO 2 or auxiliary substances.

9. Use of one or more germ-specific oligonucleotide(s) according to one of claims 1 to 7 or of a kit according to claim 8 for detection of the presence or absence of *Pseudomonas aeruginosa* strains.

10. Use according to claim 9, **characterised in that** nucleic acid hybridisation and/or nucleic acid amplification is/are carried out.

11. Use according to claim 10, **characterised in that**, as nucleic acid amplification, a polymerase chain reaction is carried out using SEQ ID NO 2 and SEQ ID NO 3 or SEQ ID NO 3 and SEQ ID NO 5 as primers.

12. Use according to one of claims 9 to 11, **characterised in that** bacteria to-be-detected are differentiated from bacteria not-to-be-detected on the basis of differences in the genomic DNA and/or RNA at at least one nucleotide position in the region of a germ-specific oligonucleotide according to one of claims 1 to 7.

13. Use according to claim 12, **characterised in that** the differentiation is carried out on the basis of differences in the region of an germ-specific oligonucleotide according to claim 1.

## Revendications

1. Oligonucléotide spécifique à un germe pour la détection des souches de *Pseudomonas aeruginosa,* **caractérisé en ce qu'**il a une longueur de 10 à 30 nucléotides et a une identité d'au moins 90 % avec la SEQ ID N°1 dans le domaine des positions nucléotidiques 12 à 131 ; la SEQ ID N°3 ; la SEQ ID N°4 ; la SEQ ID N°5 ; ou la séquence complémentaire respective.

2. Oligonucléotide spécifique à un germe selon la revendication 1, **caractérisé en ce que** l'oligonucléotide est la SEQ ID N°1 dans le domaine des positions nucléotidiques 12 à 131 ; la SEQ ID N°3 ; la SEQ ID N°4 ; la SEQ ID N°5 ; ou la séquence complémentaire respective.

3. Oligonucléotide spécifique à un germe selon l'une des revendications précédentes, **caractérisé en ce qu'**il est simple brin ou double brin.

4. Oligonucléotide spécifique à un germe selon l'une des revendications précédentes, **caractérisé en ce qu'**il se présente sous forme
(i) d'ADN ou
(ii) d'ARN correspondant à (i) ou
(iii)d'ANP.

5. Oligonucléotide spécifique à un germe selon la revendication 3 à 4, **caractérisé en ce que** l'oligonucléotide spécifique à un germe est modifié **en ce que** ou modifié en plus en ce qu'il présente comme marqueurs, d'une manière connue en soi pour les procédés de détection analytiques, un ou plusieurs groupes radioactifs, groupes colorés, groupes fluorescents, groupes pour l'immobilisation sur une phase solide et/ou groupes pour une réaction indirecte ou directe et/ou groupes se modifiant d'une autre manière ou modifiés, ayant une structure similaire aux acides nucléiques.

6. Oligonucléotide spécifique à un germe selon la revendication 5, **caractérisé en ce qu'**il présente des groupes pour une réaction enzymatique indirecte ou directe.

7. Oligonucléotide spécifique à un germe selon la revendication 5 ou 6, **caractérisé en ce qu'**il présente des groupes pour une réaction indirecte ou directe à l'aide d'anticorps, d'antigènes, d'enzymes et/ou de substances avec une affinité pour les enzymes des complexes enzymatiques.

8. Kit pour la détection de *Pseudomonas aeruginosa*, **caractérisé en ce qu'**il contient un ou plusieurs oligonucléotides spécifiques à un germe selon l'une des revendications précédentes et le cas échéant un oligonucléotide de la SEQ ID N°2 ou des auxiliaires.

9. Utilisation d'un ou de plusieurs oligonucléotides spécifiques à un germe selon l'une des revendications 1 à 7 ou d'un kit selon la revendication 8 pour la détection de la présence ou de l'absence de souches de *Pseudomonas aeruginosa.*

10. Utilisation selon la revendication 9, **caractérisée en ce que** l'on réalise une hybridation des acides nucléiques et/ou une amplification des acides nucléiques.

11. Utilisation selon la revendication 10, **caractérisée en ce que** l'on réalise une réaction en chaîne polymérase à titre d'amplification des acides nucléiques, où l'on met en oeuvre comme amorces les SEQ ID N°2 et SEQ ID N°3 ou SEQ ID N°3 et SEQ ID N°5.

12. Utilisation selon l'une des revendications 9 à 11, **caractérisée en ce que** l'on fait la distinction entre les bactéries à détecter et les bactéries qui ne sont pas à détecter à l'aide des différences existant entre les ADN et/ou ARN génomiques sur au moins une position nucléotidique dans le domaine d'un oligonucléotide spécifique à un germe selon l'une des revendications 1 à 7.

13. Utilisation selon la revendication 12, **caractérisée en ce que** l'on fait la distinction à partir de différences existant dans le domaine d'un oligonucléotide spécifique à un germe selon la revendication 1.
